# EUROPEAN PATENT APPLICATION

(11) **EP 1 842 511 A1**
(43) Date of publication of application: **10.10.2007**
(21) Application number: 07006953.9
(22) Date of filing: 03.04.2007
(51) Int. Cl.: A61F 7/00

(54) **Methods and compositions for treating non-nueropathic pain**

(30) Priority: 04.04.2006 US 789396 P; 12.04.2006 US 791253 P
(71) Applicant: Galer, Bradley S., Dr., West Chester, PA 19382-6971 (US)
(72) Inventor: Galer, Bradley S., Dr., West Chester, PA 19382-6971 (US)
(74) Representative: Duxbury, Stephen

(57) **Abstract**

Methods are provided for treating a subject for non-neuropathic pain. Aspects of the methods include applying heat to a tender area of a skin surface of a subject in a manner sufficient to treat the subject for the non-neuropathic pain. Embodiments include applying focused heat to the skin surface of a subject by using a hand-held heating device. The subject methods find use in the treatment of a variety of different types of non-neuropathic pain conditions, such as fibromyalgia and myofascial pain syndrome. Also provided are kits for use in practicing the subject methods.

## Description

### BACKGROUND

Millions of people across the world experience some form of acute or chronic non-neuropathic pain. Non-neuropathic pain most commonly is musculoskeletal in origin, affecting the soft tissues, which include muscles, ligaments, tendons, and bones. Many causes of musculoskeletal pain can be attributed to the wear and tear of daily activities which leads to muscle tissue damage or trauma to a localized area. For example, auto accidents, falls, fractures, sprains, dislocations and direct blows to the muscle may lead to musculoskeletal pain. Additional sources of pain include postural strain, repetitive movements, overuse, and prolonged immobilization. Other forms of pains in the soft tissues, such as fibromyalgia, which may be idiopathic in nature as the underlying pathophysiology remains unknown, though the pain is experienced in the soft tissues.

The number of people affected by non-neuropathic pain is increasing rapidly world-wide. In fact, musculoskeletal conditions are the most common causes of severe chronic pain and physical disability in the United States, and probably elsewhere in the world. Examples of common musculoskeletal conditions where the musculoskeletal system is thought to be involved in the development of pain, include but are not limited to, myofascial pain syndromes, whiplash associated disorders, piriformis muscle syndrome, soft tissue pain syndromes, inflammatory muscle pain, temporomandibular disorders, Sjögren's syndrome, chronic plantar fasciitis, osteoarthritis, rheumatoid arthritis, unilateral shoulder myalgia, carpal tunnel syndrome, chronic neck, shoulder, or back pain, repetitive strain injury, and exercise-induced muscle injury.

Chronic myofascial pain syndrome (CMP) is a painful musculoskeletal condition which can affect any skeletal muscle in the body. CMP is characterized by the development of myofascial trigger points that are locally tender when active and can also refer pain through specific patterns to other areas of the body. Trigger points are usually associated with a taut band of muscle tissue. Typically, a trigger point, when pressed upon, will cause the afflicted individual to feel pain both at the trigger point itself and also at locations distant to the trigger point. This is what is considered as "referred pain".

Because trigger points refer pain to other areas of the body, CMP is often the cause of most common aches and pains. For instance, many people diagnosed with carpal tunnel syndrome are actually experiencing pain caused by a trigger point in a muscle located within the armpit (subscapularis). Additionally, in the head and neck region, myofascial trigger points can manifest as tension headaches, tinnitus, temporomandibular joint pain, eye symptoms and torticollis. In the lower extremities, trigger points cause pain in the quadriceps and calf muscles and may lead to a limited range of motion in the knee and ankle. Myofascial trigger point hypersensitivity in the glueteus maximus and gluteus medius often produces intense pain in the lower back region and, in fact, are believed to be the most common cause of both acute and chronic low back pain.

Fibromyalgia is one of the most common chronic pain disorders where pain is present in soft tissues throughout the body, though its etiology is unclear. Fibromyalgia syndrome (FM) is a condition of widespread muscular pain and fatigue. Fibromyalgia is known to affect women more than men and has been found in all age groups ranging from young children to senior citizens, although most people generally develop symptoms during their 20's or 30's. People with fibromyalgia have "tender points" on the body which are specific places on the neck, shoulders, back, hips, arms and legs that hurt when pressure is applied to them.

In patients suffering from FM; the pain is intense, widespread and chronic. All parts of the body feel the pain with varying intensity. FM pain has been described as deep muscular aching, throbbing, twitching, stabbing and shooting pain that is constant and encompasses every aspect of a patient's daily life. Additional symptoms include neurological effects such as numbness, tingling and burning. Factors that provide additional irritation for fibromyalgia patients include cold/humid weather, non-restorative sleep, physical and mental fatigue, excessive physical activity, physical inactivity, anxiety and stress.

In patients suffering with non-neuropathic pain conditions, e.g., musculoskeletal conditions such as CMP or FM, there are no "gold standard" treatments that work for every patient. Currently, no drug has a FDA approved indication for the treatment of either condition. Some of the more common medications prescribed for CMP and FM consist of nonsteroidal anti-inflammatory (NSAID) medications and medications to increase the body's level of serotonin and norepinephrine (neurotransmitters that modulate pain, sleep, and mood,).

Probably one of the most common treatments have included injections with anesthetic or anti-inflammatory medications in or around the painful sites, so-called "trigger point injections" and "tender point injections" for CMP and FM, respectively. A variety of different techniques of trigger point injections can be used including injecting a small amount of local anesthetic (like lidocaine) directly into the trigger point; injecting a mixture of local anesthetic with a corticosteroid; injecting Botulinum Toxin (especially into trigger points); and inserting an acupuncture needle directly into the trigger point without any medication injected (called "intramuscular stimulation" or "dry needling"). In addition to relieving pain, trigger point injections can significantly increase mobility in the affected muscle, ligament and tendons. All of these methods have some anecdotal evidence showing that they can relieve myofascial pain and fibromyalgia pain for days or weeks or months in patients. The exact mechanism of trigger point injections or tender point injections is not known though it is believed they cause the abnormal muscle, tendon, and ligament to restore its function to a normative nonspastic state.

For most painful non-neuropathic disorders, including CMP and FM, an active exercise program that includes muscle strengthening and stretching is currently believed to be an important element to treatment. Therapies that alleviate the ongoing pain, such as trigger point injections and tender point injections, can make an important contribution by allowing the patient to partake in such an active physical therapy program and improve the chances of successful treatment.

The numbers of people affected by non-neuropathic pain are set to rise over the next few decades due to the increasing age of our population. Musculoskeletal pain disorders and fibromyalgia have an immense toll on our society, affecting not only the patients, but also their families, employers, and society as a whole in terms of medical costs and loss of productivity.

### SUMMARY

Methods are provided for treating a subject for non-neuropathic pain. Aspects of the methods include applying focused heat directly to a tender area within the soft tissues, e.g., a trigger point or tender point, on a skin surface of a subject in a manner sufficient to treat the subject for the non-neuropathic, e.g., musculoskeletal or fibromyalgia pain. Embodiments include applying heat to the skin surface of a subject by using a hand-held heating device. In some aspects, the hand-held heating device is disposable. In certain embodiments, the patient may apply a topical medication, such as a topical pain medication, to the tender area in conjunction with heat application, e.g., prior to heat application. The subject methods find use in the treatment of a variety of different types of non-neuropathic pain conditions, such as myofascial pain syndrome and fibromyalgia. Also provided are kits for use in practicing the subject methods.

### BRIEF DESCRIPTIONS OF THE DRAWINGS

The figures shown herein are not necessarily drawn to scale, with some components and features being exaggerated for clarity.

Figure 1 is a cross-section view of a first embodiment of a heating device according to an embodiment of the invention.

Figure 2 is an electrical schematic illustrating the operation of the device shown in Figure 1.

Figure 3 is a cross-section view of another embodiment of a heating device which may be used with the subject methods.

Figure 4A illustrates an example of a myofascial trigger point located within a taut band of muscle fibers and its corresponding tender area.

Figure 4B is an expanded view of the myofascial trigger point depicted in Figure 4A.

Figure 5 provides the 18 fibromyalgia tender points which comprise the American College of Rheumatology's criteria for fibromyalgia.

Figures 6A to 7C provide examples of referred pain from myofascial trigger points.

### DETAILED DESCRIPTION

Methods are provided for treating a subject for non-neuropathic pain. Aspects of the methods include applying focused heat to a musculoskeletal tender area, such as a trigger point or tender point, of a skin surface of a subject in a manner sufficient to treat the subject for the non-neuropathic pain. Embodiments include applying heat to the skin surface of a subject by using a hand-held heating device. Embodiments of the invention further include applying a topical medication to the tender area in conjunction with focused heat, e.g., prior to the application of focused heat. The subject methods find use in the treatment of a variety of different types of non-neuropathic pain conditions, such as myofascial pain syndrome and fibromyalgia. Also provided are kits for use in practicing the subject methods.

Before the present invention is described in greater detail, it is to be understood that this invention is not limited to particular embodiments described, as such may vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention. Any recited method can be carried out in the order of events recited or in any other order which is logically possible.

As summarized above, the subject invention provides methods and compositions for treating a subject for musculoskeletal pain. In further describing aspects of the invention, aspects of the subject methods are reviewed first in greater detail, followed by a review of other aspects of the invention, e.g., kits, as well as applications in which the present methods and kits find use.

### METHODS

Aspects of the invention include topically applying heat to a tender area, such as a trigger point or tender point, of a subject in a manner sufficient to treat the subject for non-neuropathic pain. In certain embodiments, the non-neuropathic pain is musculoskeletal pain. By "musculoskeletal pain" is meant any pain felt within the muscles, ligaments, tendons, and bones of a subject. In aspects of the invention, "musculoskeletal pain" describes the pain associated with the musculoskeletal conditions described above.

By "tender area" is meant an area on the skin surface that is more sensitive when pressure is applied to it as compared to other areas of the body. For example, a tender area is a localized area on the skin surface that produces pain when pressed. Commonly these tender areas on the skin overlie soft tissues, muscles, tendons, and ligaments, or bone, such as the sternum. See e.g., Alvarez et al., American Family Physician (2002) 65: 653-660.

In CMP, a latent trigger point does not cause spontaneous pain, but may restrict movement or cause muscle weakness. A CMP patient may complain of limited muscle movement, stiffness, or weakness and may only become aware of pain originating from a latent trigger point when pressure is applied directly over the trigger point. In addition, when firm pressure is applied over the trigger point in a snapping fashion perpendicular to the muscle, a "local twitch response" is often elicited. A local twitch response is a transient visible or palpable contraction or dimpling of the muscle and skin as the tense muscle fibers (taut band) of the trigger point contracts when pressure is applied; typically pressure is applied by gently pressing the index finger over the trigger point. A classic trigger point is defined as the presence of discrete focal tenderness located in a palpable taut band of skeletal muscle, which produces both referred pain and a local twitch response.

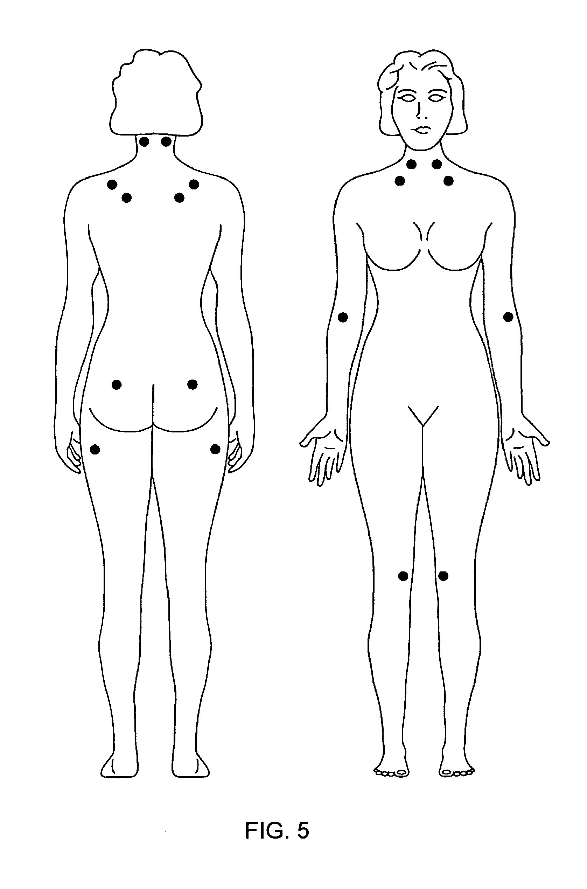

In certain embodiments, in particular with respect to the fibromyalgia tender points, topical areas are considered to be "tender" when an individual feels pain when palpated with an approximate force of 4kg (roughly the amount of pressure needed to change the color of the skin). In contrast to trigger points of CMP, tender points of fibromyalgia are associated with pain at the site of palpation only, are not associated with referred pain, and occur in the insertion zone of muscles, not in taut bands in the muscle belly. In order to diagnose fibromyalgia, according to the American College of Rheumatologists Diagnostic Criteria, patients must have pain in all four quadrants of their body and have tenderness in at least 11 of the 18 defined tender points when 4 kg of pressure are applied: (evaluated on both sides of the body) attachment of neck muscles at the base of the skull; midway between neck and shoulder; muscle over upper inner shoulder blade; 2 cms below side bone at elbow; upper outer buttock; hip bone; just above knee on inside; lower neck in front; edge of upper breast bone. See e.g., FIG. 5.

By "applying *focused* heat" is meant that heat is applied directly and only to a defined tender area of a skin surface of a subject in a manner sufficient to treat the subject for musculoskeletal pain. In certain embodiments, the heat is applied solely to the tender area, and not to locations adjacent the tender area; the area of heat from the device may be as small as the tip of a ballpoint pen to as large as a quarter. In certain embodiments, if heat is applied to skin locations adjacent the tender area, the surface area of the non-tender area surface does not exceed that of the surface area of the tender area, and may be 80%, 60%, 50%, 40% or less than the surface area of the tender area. As such, focused heat is applied to the tender area, where by "focused" is meant that the heat is not applied to skin locations adjacent the defined tender area.

In certain embodiments, "subjects" of the invention are mammals, including, but not limited to: dogs, cats, horses, and "humans." Aspects of the invention include the treatment of humans suffering from musculoskeletal pain with the subject methods, where the subject methods are particularly suited for use in the treatment of humans suffering from non-neuropathic pain conditions, such as musculoskeletal pain and related conditions, fibromyalgia pain, etc., as described above. In certain embodiments, the subject is one that is known to be suffering from musculoskeletal pain, where embodiments of the methods may include first diagnosing the presence of musculoskeletal pain in the subject followed by treatment of the pain, e.g., as described below.

Embodiments of the invention include a subject employing a heating device to apply focused heat according to the present methods. As reviewed in greater detail below, embodiments of the invention provide devices which apply focused heat to a tender area, e.g., latent trigger point area, of a skin surface of a subject for the treatment of musculoskeletal pain.

Any convenient device that provides the requisite heat to a tender area of a skin surface of a subject may be employed. In certain embodiments, the device may be a hand-held portable device. In certain aspects of the present invention; heat is applied to the skin surface of a subject at a specified temperature which is effective for treating musculoskeletal pain but does not burn the subject's skin.

In certain embodiments, the heating devices include one or more heating elements. As such, some embodiments include at least one source of heat but may include multiple heating sources. The heating devices employed in the subject methods may include any of the various types of heaters known in the art. Accordingly, as used herein, a "heating source" is meant any heating element capable of generating the amount of heat appropriate for the methods of the present invention which can be used as a heat source in the devices which apply heat to a tender area of a subject's skin surface for the treatment of musculoskeletal pain. In certain embodiments, the heater may be a semiconductor device which includes a transistor, a cartridge heater, a lamp, light bulb, or a foil-type heater attached to a thermal mass. In other embodiments, the heat source may be a heat conducting liquid or a chemical solution or any other type of heat source which is capable of generating the desired temperature for treating musculoskeletal pain according to the present methods.

Certain embodiments of the device include a heat application surface which contacts the tender area of a skin surface of a subject. In certain embodiments, the heating element of the device is on the heat application surface such that the heating element directly contacts the skin surface of the subject. The heat application surface may be made of a number of different materials such as a heat conductive metal in conjunction with a magnetic-induction type heater. The surface may alternatively be covered by a non-heat-conducting coating or material which reduces the conduction speed of the heat applied to the skin providing for the gradual application of heat to the user's skin surface.

In certain aspects, the heating device includes a temperature selector which enables users to directly select one best effective temperature for the heater. In other aspects, the temperature selector provides for selection of two or more predetermined temperatures for example, different ranges of temperatures. In certain embodiments, the temperature selector is a rotor with a pointer to indicate which temperature is selected for treating musculoskeletal pain. In such embodiments, methods may include selecting the temperature that is to be employed.

The device may also include a thermostat/thermometer which measures the temperature of the tender area on the skin surface of the subject prior to the application of heat and in turn, applies a specific amount of heat sufficient to raise the temperature of the tender area to the selected temperature.

In some embodiments, the heating devices may also include a temperature indicator which signals to the subject that the tender area has reached the selected temperature. For example, the temperature indicator may be a light or color, which flashes from the device. In such embodiments, methods may include a step of discontinuing heat application when the temperature indicator provides such a signal. In other embodiments, the device is programmed to immediately shut off once the tender area has reached the selected temperature.

Additionally in some aspects, the heating devices are able to account for the differences in each individual subject's skin surfaces and accordingly adjust the amount of heat applied to the tender area. For example, in certain aspects, a thermometer within the heating device measures the temperature of the tender area on the skin surface of the subject prior to the application of heat and during the application of heat until the specified temperature of the skin surface has been reached. The application of heat is specific to the individual's skin surface because the temperature is continuously being measured by the heating device and stops once the specified temperature has been reached.

The heating device may include a self-contained power supply for portability, e.g., batteries. However, in other embodiments, the device includes a cord to be plugged into a normal household utility outlet.

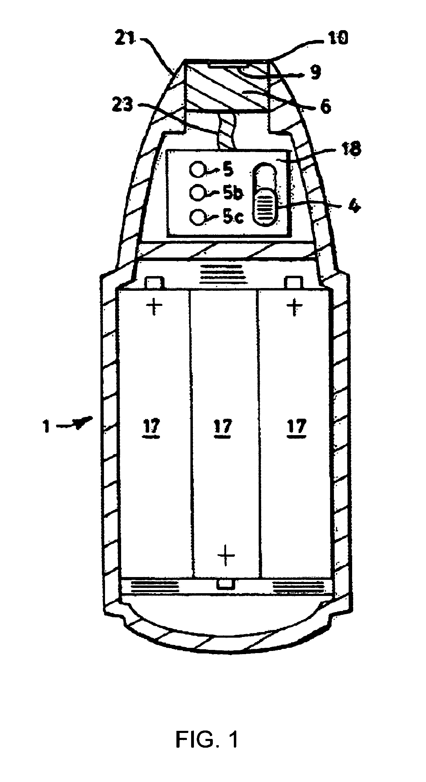

In the embodiment illustrated in Figure 1, the heating device includes a housing (1) and a temperature selector (4) capable of selecting temperature in 1 degree-centigrade increments to within one-half-degree centigrade. The device includes a light indicator (5) which illuminates when the power is on and additional light indicators (5b and 5c), which each correspond to different pre-selected temperatures. These indicators (5b and 5c) illuminate once the selected temperature has been reached. The device includes a heat application surface (10) which applies heat to a tender area on the skin surface of an individual for treating musculoskeletal pain by directly contacting the user's tender area. Adjacent to the heat application surface is a transducer or thermostat (7), which allows the temperature of the user's skin surface to be detected while heat is being applied. As shown in Figure 1, batteries are located at the bottom portion of the housing (1) so that the device includes a self-contained power supply.

In certain aspects of the invention, the electronics used to control the device are mounted on a circuit board (18) located in the housing as shown in Figure 1. The circuit board contains control electronics which supplies current to the heating element through cable (21) when the temperature sensed is below the temperature commanded by temperature selector (4). If the temperature reaches or exceeds the temperature commanded, the current is discontinued. The control electronics provide a smooth response profile (i.e. temperature vs. time), with a minimum of overshoot, to a precision of plus or minus one-half degree centigrade.

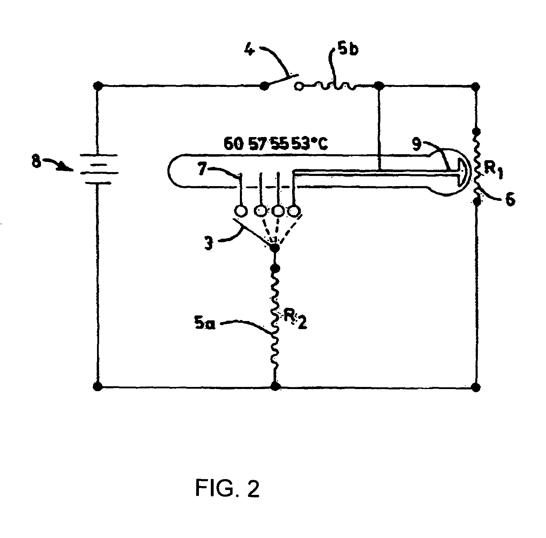

Figure 2 provides an electrical schematic which illustrates the operation of the embodiment in Figure 1. The power source in the form of a battery (8), is connected through switch (4) in series with indicator light (5b) to the temperature transducer (9) and heater (6). The multi-position switch (3) selects one of several contacts which detect different positions along the transducer corresponding to different temperatures. When the selected temperature is reached, the transducer makes an electrical connection with the rest of the system, allowing the "READY" indicator (5a) to illuminate. The temperature transducer is a temperature probe (9) filled with mercury. When the heater is at lower than the selected temperature, the thermostat allows the maximum current to go through the heating element. When the heater reaches the selected temperature, the mercury will serve as a conductor to divide and therefore reduce the heater current, thereby reducing it sufficiently to maintain the selected temperature.

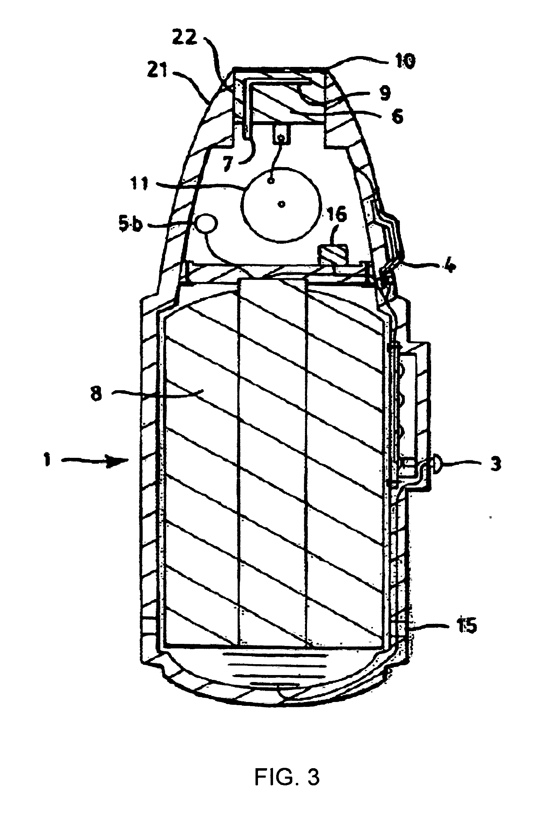

Figure 3 depicts a second embodiment of a heating device for use with the subject methods. In this embodiment, the heat transfer surface/heater combination is slidingly mounted in a channel (22) within the contact end of the device. The heater has an extended position, in which the heater is in contact with the skin of the user and a retracted position, in which the heater is withdrawn within the channel. The heater is driven between its extended and retracted positions by a positioning mechanism (11), e.g., a motor/crank combination or a solenoid.

In certain embodiments, the heating device for use with the subject methods is a disposable heating device. By "disposable" is meant that the device may be "disposed of' or "thrown away" after the heating device is used to topically apply heat to a tender area on the skin surface of a subject in a manner sufficient to treat the subject for non-neuropathic pain. In certain aspects, the disposable heating device is a single-application heating device such that the device is used to topically apply heat to a tender area on the skin surface of a subject once before the user disposes of the heating device.

In certain aspects, the disposable heating device has a restricted number of applications. By "restricted number of applications" is meant that the disposable heating device may be used to apply heat a specific number of times to at least one tender area on the skin surface of a subject, i.e., applications, before the subject disposes of the heating device. For example, the number of applications may range from about 1 to about 500, such as from about 10 to about 250, and including from about 25 to about 100. In some embodiments, the disposable heating device applies heat to "at least one tender area" before the user disposes of the heating device, where in certain embodiments, the number of tender areas ranges from about 1 to about 1000, such as from about 10 to about 500, and including from about 25 to about 250.

The disposable heating device in certain aspects is used to topically apply heat to at least one tender area on the skin surface of a subject a plurality of times over a given time period before the subject disposes of the heating device. In certain embodiments, the period of time may be about 10 minutes or more, e.g., 30 minutes or more, e.g., 60 minutes or more, e.g., 2 or more hours, e.g., 6 hours or more, e.g., 12 hours or more, e.g., 1 day or more, e.g., 7 days or more, e.g., 14 days or more, where in certain embodiments the period of time does not exceed about 30 days. For example, the time period may range from about 10 minutes to about 30 days, e.g., from about 30 minutes to about 7 days.

Aspects of the embodiment include the repetitive movement of the heater against the skin of the user which is controlled by the temperature selection/detection. The rate of movement is controlled by a first switch which controls the rate at which the heater moves against the skin, in seconds per cycle and a second switch, which controls the duration of the application, in seconds.

In some embodiments, a grid is located at the contact end of the heating device which contacts the skin surface of the user during application. The heat application surface contains raised projections which mate with the grid and protrude through the grid when the heater is in the extended position. As such, these projections contact the skin surface and provide a safety mechanism when the heating element is retracted. Further, the temperature detector can be located in the grid itself and provide a more accurate measure of the user's skin temperature because it directly contacts the user's skin surface.

In certain embodiments, heat at a specified temperature is applied to a tender area of the skin surface of a subject in a manner sufficient to treat the subject for non-neuropathic musculoskeletal pain. As reviewed above, in certain embodiments heat is applied substantially solely, if not solely, to the tender area to the exclusion of non-tender are skin regions. While the "specified temperature" applied in a given embodiment may vary, in certain representative embodiments the temperature of the heat applied to a tender area of a skin surface of a subject by practice of the subject methods ranges from about 40°C to about 60°C such as from about 45°C to about 55°C, including from about 47°C to about 53°C. The application of heat to a tender area of a skin surface of a subject may last for an extended period of time, e.g., from about 10 seconds to about 300 seconds, such as from about 30 seconds to about 180 seconds, including from about 60 seconds to about 120 seconds. In certain embodiments, applying heat according to the subject methods includes applying heat to more than one tender area on the skin surface of the subject. In other embodiments, heat is applied to at least two tender areas multiple times. Depending on the patient's pain condition and number of tender areas, e.g., trigger points or tender points, the number of areas treated with the device sequentially during one treatment session may be only one area or may exceed 20 areas.

Depending on the depth of the tender area in the soft tissues underlying the skin, the person applying the focused heat, whether it be the patient himself or another person, may need to apply pressure on the heated device when on the skin in order to optimize treatment. This pressure may need to be½ kg to 10 kg force.

The present methods may topically apply heat to a tender area on the skin surface of a subject where the tender area may be on any skin surface site of the subject's body, including but not limited to: arms, legs, face, head, neck, back, buttocks, shoulders, etc. In certain embodiments of the present methods, the heat may be applied to one or more tender areas located at various sites of the subject's skin surface for treating musculoskeletal pain.

The surface area of the tender area of the subject's skin surface upon which heat is applied may vary depending on a variety of factors such as the particular musculoskeletal pain being treated, the location of the tender area on the subject's body, the length of time the heat is applied, or the temperature of the heat applied to the tender area, etc. In certain embodiments, the target tender area may have be roughly circular and have a diameter ranging from about .1 to about 3 cm, such as from about .2 to about 2 cm, including from about .5 to about 1.5 cm.

In practicing the subject methods, heat may be topically applied to a tender area once or a plurality of times over a given time period, where the application schedule may be hourly, daily, weekly, biweekly, etc. For example, heat may be applied to a tender area on the skin surface of a subject two or more times a day, two or more times a week, etc.

The application of heat to a tender area of a skin surface of a subject is sufficient to provide for the desired reduction in at least one aspect of musculoskeletal pain, e.g., frequency and/or intensity of the pain. In addition, the application of heat to a tender area of a skin surface of a subject may be sufficient to provide for the desired improved mobility and range of motion. The heat is maintained in place for a period of time sufficient for the desired reduction in pain or improved mobility to occur. The particular duration of time during which the heat is maintained at the tender area depends on a variety of factors such as, but not limited to, the nature of the pain, the location of the tender area on the subject's body, the sensitivity of the subject to the applied heat, etc.

In certain embodiments, the present methods may be repeated one or more times so that heat is applied at multiple times to a tender area of a skin surface of a subject. For example, heat may be applied every 4 hours. In certain embodiments, during the course of a day, the number of distinct heat applications may range from about 1 time to about 10 times or more per day, Embodiments of the invention include gradually decreasing or increasing the number of applications of heat over time by decreasing or increasing the number of times heat is applied to a tender area of a skin surface of a subject. Other embodiments include gradually decreasing or increasing the temperature of the heat applied to the tender areas of a skin surface of a subject for treating musculoskeletal pain.

An application of heat to a tender area of a skin surface of a subject according to aspects of the invention results in treating non-neuropathic pain which is local, referred, or a combination of both. Accordingly, in certain embodiments the subject methods include applying heat at an effective temperature for an effective amount of time to a tender area of a skin surface of a subject in order to treat the subject for CMP or FM pain. In some aspects of the invention, the pain condition is localized pain. In other aspects, the treated pain is referred in which the afflicted individual feels pain at locations other than the tender area, e.g., trigger point or tender point. In certain embodiments, the non-neuropathic pain is localized and referred.

As such, the subject methods at least provide for improvement in the condition being treated. Improvements may be assessed by at least an amelioration of the pain for a period of time and includes situations where the musculoskeletal pain is completely inhibited, e.g., prevented from happening, or stopped, i.e., terminated, such that the subject no longer suffers at least for a period of time. As such, application of heat to a tender area of a skin surface of a subject as described above results in at least an amelioration or reduction in the magnitude and/or frequency of musculoskeletal pain, including a complete cessation or removal of the pain for a period of time, e.g., for about 1 hour or more, e.g., a period of time that may be about 5 hours or longer, e.g., about 10 hours or longer in certain embodiments, e.g., at about 24 hours or longer, e.g., about 2 days, or longer.

In certain embodiments, additional treatment modalities may be employed in conjunction with the application of heat. For example, in certain embodiments, topical pain medications may be employed in conjunction with the use of heat, as described above, where by in conjunction is meant that the topical medication is applied either before or after the application of heat. The topical pain medications can be any of a variety of different types of formulations, including but not limited to: a patch, gel, cream, suave, or solutions. Topical pain medications of interest include, but are not limited to: local anesthetics, such as lidocaine, benzocaine, prilocaine, and novocaine; nonsteroidal anti-inflammatory drugs, such as ibuprofen, ketoprofen, diclofenac, naproxen, and indomethacin; COX-2 inhibitory drugs such as celecoxib and valdecoxib; opioid drugs such as morphine, oxycodone, oxymorphone, methadone, hydrocodone, hydromorphine and codeine; capsaicin and capsaicin analogs; and menthol and menthol analogs. In certain embodiments, the patient may first apply a topical medication, such as a local anesthetic or nonsteroidal anti-inflammatory composition, directly to the skin overlying the tender point and after a period of time, for example ranging from 5 minutes to several hours, apply heat to the skin surface of a subject, e.g., by using a hand-held heating device.

### UTILITY

Applications of the subject methods include applying heat to a tender area of a skin surface of a subject in a manner sufficient to treat a non-neuropathic pain condition, where the methods may or may not include application of heat in conjunction with other treatment modalities, e.g., application of a topical medication, etc., as described above. For example, the subject methods find use in the treatment of a variety of different musculoskeletal conditions, including but not limited to, myofascial pain syndromes, whiplash associated disorders, piriformis muscle syndrome, soft tissue pain syndromes, inflammatory muscle pain, temporomandibular disorders, Sjögren's syndrome, chronic plantar fasciitis, osteoarthritis, rheumatoid arthritis, unilateral shoulder myalgia, carpal tunnel syndrome, chronic neck, shoulder, or back pain, repetitive strain injury, exercise-induced muscle injury, and fibromyalgia. A variety of subjects are treatable according to the present methods. In certain embodiments, the subjects are humans where the subject methods are particularly suited for use in the treatment of humans suffering from musculoskeletal pain and musculoskeletal conditions, as described above. The embodiments also apply to the treatment of non-human mammals.

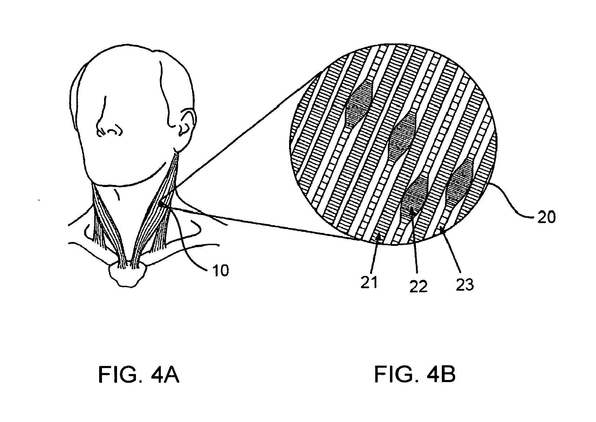

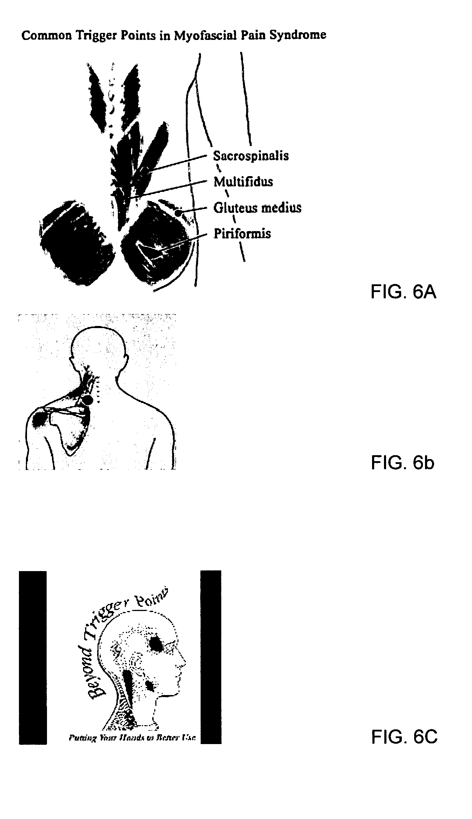

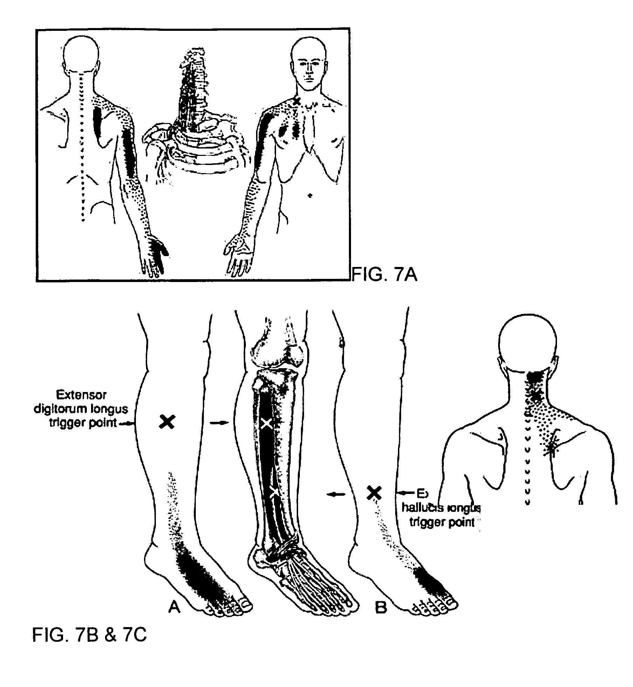

In certain embodiments, the musculoskeletal condition being treated may be chronic myofascial pain syndrome. In such embodiments, heat is applied to a tender area of a skin surface of a subject in a manner to treat MPS. When treating MPS, the tender areas on the subject's skin surface correspond to myofascial trigger points. By "myofascial trigger point" is meant a discrete, focal, hyperirritable spot located in a taut band of skeletal muscle. For example, Figures 4A and 4B illustrate a sternocleidomastoid muscle(10) and its corresponding muscle fibers (20). Some of the muscle fibers are in a normal resting state (21) and some are contracted (22). However, a trigger point (23) is a tight knot that forms when the muscle fibers are in a contracted state and fail to return to the relaxed state. Because a trigger point is most commonly chronically contracted, it essentially blocks the flow of blood in the immediate area. One theory is that this results in oxygen starvation and an accumulation of metabolic waste products that irritate the trigger point in which the subject's body responds by sending out pain signals. Applying pressure to a trigger point may elicit localized pain, i.e. the corresponding skin surface above the trigger point, and/or referred pain such that the pain is not felt at the trigger-point origin but instead at a different location of the subject's body, which may also radiate outward from that different location. Examples of referred pain from myofascial trigger points are seen in Figures 6A to 7B. In embodiments of the present invention where myofascial pain syndrome is treated,heat is applied to a tender area on the skin surface of a subject which corresponds to a myofascial trigger point for treating CMP. In certain embodiments, a hand-held portable device applies heat to the tender area which corresponds to a myofascial trigger point.

In other embodiments of the invention, the non-neuropathic pain condition may be fibromyalgia syndrome. In such embodiments, the tender area on the skin surface of a subject corresponds to a fibromyalgia tender point. Tender points produce pain where pressed, but unlike myofascial trigger points, do not refer pain to some other part of the body. Figure 5 illustrates the 18 tender points that comprise the American College of Rheumatology criteria for fibromyalgia; patients may have more than the 18 fibromyalgia tender points described by the American College of Rheumatology. In order to meet the criteria for the diagnosis of fibromyalgia according to certain diagnostic metrics, a patient must have had widespread pain for at least three months and must have tenderness at 11 or more of the 18 specific tender points. In treating fibromyalgia syndrome according to the present methods, heat is applied to a tender area on the skin surface of a subject corresponding to a fibromyalgia tender point. In certain embodiments, a hand-held portable device applies heat to the fibromyalgia tender point.

### KITS

Also provided are kits for practicing the present methods. The present kits may vary greatly in regards to the components that are included in the kit. The present kits at least include a heating device for use in practicing the present methods. The heating device may be any suitable device for applying heat to a tender area of a skin surface of a subject for treating musculoskeletal pain as described above. In certain embodiments, the heating device is a portable hand-held device as described above.

The present kits may also include medications that interact with norepinephrine and/or serotonin, such as but not limited to serotonin reuptake inhibitors, amitriptyline, fluoxetine, cyclobenzaprine, imipramine, clomipramine, sertraline, nefazodone, venlafaxine, trazodone, bupropion, nortriptyline, doxepin, and milnacipran, or neuromodulators, such as pregabalin. In other aspects, the kit may include an analgesic such as but not limited to acetaminophen, tramadol, codeine, morphine, darvon, fentanyl, diflunisol, oxycodone, oxymorphone, and nonsteroidal anti-inflammatory agents.

Other kits may include topical medications in a patch, gel, cream, suave, or solution to be applied to the trigger point or tender point prior to the application of heat, where agents of interest include but are not limited to: local anesthetics, such as lidocaine, benzocaine, prilocaine, and novocaine; nonsteroidal anti-inflammatory drugs, such as ibuprofen, ketoprofen, diclofenac, naproxen, and indomethacin; COX-2 inhibitory drugs such as celecoxib and valdecoxib; opioid drugs such as morphine, oxycodone, oxymorphone, methadone, hydrocodone, hydromorphine and codeine; capsaicin and capsaicin analogs; and menthol and menthol analogs.

The subject kits may include instructions for applying heat to a tender area of a skin surface of a human for treating musculoskeletal pain. The instructions may be recorded on a suitable recording medium or substrate. For example, the instructions may be printed on a substrate, such as paper or plastic, etc. As such, the instructions may be present in the kits as a package insert, in the labeling of the container of the kit or components thereof (i.e., associated with the packaging or sub-packaging) etc. In other embodiments, the instructions are present as an electronic storage data file present on a suitable computer readable storage medium, e.g. CD-ROM, diskette, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, this means for obtaining the instructions is recorded on a suitable substrate.

The following examples are offered by way of illustration and not by way of limitation.

### EXPERIMENTAL

The following examples are put forth so as to provide those of ordinary skill in the art with a complete disclosure and description of how to make and use the present invention, and are not intended to limit the scope of what the inventors regard as their invention. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

Treatment protocols according to certain embodiments are as follows:
I. Protocols
   A. Chronic Myofascial Pain
      1. A patient with musculoskeletal pain can have CPM diagnosed by one or more several methods:
         a. Physician examination
         b. Physical Therapist evaluation
         c. Massage Therapist evaluation
         d. Self diagnosis (via gaining knowledge about CPM and self-examination); it is recommended patients have diagnoses confirmed by a trained physician
      2. Identification of trigger points by
         a. Health care provider
         b. Self-examination
      3. Use of focused heating device applied directly onto skin overlying the trigger point
         a. Patient will identify optimal temperature, duration of heat, and pressure in which to apply to stimulus for each trigger point
      4. Upon cessation of heat treatment, patient will gently stretch the involved muscle for several minutes
   B. CPM with Topical Drug Applied Prior to Heat
      1. A patient with musculoskeletal pain has CPM diagnosed by one or more of several methods:
         a. Physician examination
         b. Physical Therapist evaluation
         c. Massage Therapist evaluation
         d. Self diagnosis (via gaining knowledge about CPM and self-examination); it is recommended patients have diagnoses confirmed by a trained physician
      2. Identification of trigger points by
         e. Health care provider
         f. Self-examination
      3. Application of topical medication to skin overlying trigger point region
      4. After waiting 5 - 60 minutes, use of focused heating device applied directly onto skin overlying the trigger point
      5. Patient will identify optimal temperature, duration of heat, and pressure in which to apply to stimulus for each
      6. Upon cessation of heat treatment, patient will gently stretch the involved muscle for several minutes
   C. CPM with Topical Drug Applied After Head
      1. A patient with musculoskeletal pain has CPM diagnosed by one or more of several methods:
         g. Physician examination
         h. Physical Therapist evaluation
         i. Massage Therapist evaluation
         j. Self diagnosis (via gaining knowledge about CPM and self-examination); it is recommended patients have diagnoses confirmed by a trained physician
      2. Identification of trigger points by
         k. Health care provider
         l. Self-examination
      3. Upon cessation of heat treatment, patient will gently stretch the involved muscle for several minutes
      4. After waiting 5-60 minutes after use of focused heating device applied directly onto skin overlying the trigger point, application of topical medication to skin overlying trigger point region
   D. Fibromyalgia
      1. A patient with fibromyalgia pain has the disorder diagnosed by one or more of several methods:
         m. Physician examination
         n. Physical Therapist evaluation
         o. Massage Therapist evaluation
         p. Self diagnosis (via gaining knowledge about fibromyalgia and self-examination); it is recommended patients have diagnoses confirmed by a trained physician
      2. Identification of tender points by
         a. Health care provider
         b. Self-examination
      3. Use of focused heating device applied directly onto skin overlying the trigger point
      4. Patient will identify optimal temperature, duration of heat, and pressure in which to apply to stimulus for each
   E. Fibromyalgia with Topical Drug Before Heat
      1. A patient with fibromyalgia pain is diagnosed by one or more of several methods:
         q. Physician examination
            r. Physical Therapist evaluation
            s. Massage Therapist evaluation
            t. Self diagnosis (via gaining knowledge about CPM and self-examination); it is recommended patients have diagnoses confirmed by a trained physician
      **2.** Identification of tender points by
         u. Health care provider
         v. Self-examination
      3. Application of topical medication to skin overlying tender point
      4. After waiting 5-60 minutes, use of focused heating device applied directly onto skin overlying the tender point
      5. Patient will identify optimal temperature and duration of heat stimulus for each tender point
   F. FM with Topical Drug Applied After Head
      1. A patient with musculoskeletal pain has CPM diagnosed by one or more of several methods:
         w. Physician examination
         x. Physical Therapist evaluation
         y. Massage Therapist evaluation
         z. Self diagnosis (via gaining knowledge about CPM and self-examination); it is recommended patients have diagnoses confirmed by a trained physician
      2. Identification of trigger points by
         aa. Health care provider
         bb. Self-examination
      3. Upon cessation of heat treatment, patient will gently stretch the involved muscle for several minutes
      4. After waiting 5 - 60 minutes after use of focused heating device applied directly onto skin overlying the trigger point, application of topical medication to skin overlying tender point region
II. EXAMPLES
   A. A 45 year old man who has a predisposition to developing shoulder and neck myofascial dysfunction with referred pain into the head developed moderate pain in his left shoulder and neck region, with a mild headache in his left forehead after working at a computer typing for approximately 8 hours throughout the day. He identified trigger points in his left sternocleinomastoid muscle and left trapezius muscle that when palpated were very tender and mimicked his neck and shoulder pain and caused a worsening of his left sided headache. He also noticed a decrease range of motion in his ability to turn his head. He then applied a focal heating device (ZENO), which applied approximately 50 degree centigrade heat, directly to the identified trigger points for 2.5 minutes. After application of focal heat to one trigger point in his sternocleinomastoid and one trigger point in his trapezius muscle, he experienced approximately a 75% reduction in his shoulder and neck pain and a complete alleviation of his headache pain that lasted 24 hours. He also noticed about a 50% improvement in his neck range-of-motion. Approximately 24 hours later, he began to notice the pain returning and his neck becoming stiff. He again applied the same heat stimulus to the same trigger points and his pain and movement were significantly improved without a return of his symptoms.
   B. A 43 year old woman who has a history of spasmodic torticollis and has a predisposition to developing shoulder and neck myofascial dysfunction with pain in the shoulder and neck region. She identified trigger points in her left and right trapezius muscles that when palpated were very tender and mimicked her neck and shoulder pain and caused a worsening of her pain. She also noticed a decrease range of motion in her ability to turn her head. She then applied a focal heating device (ZENO), which applied approximately 50 degree centigrade heat, directly to the identified trigger points for 2.5 minutes. After application of focal heat to one trigger point in her left trapezius muscle and to one trigger point in her right trapezius muscle, she experienced approximately a 90% reduction in her shoulder and neck pain that lasted 12 hours. She also noticed about a 75% improvement in her neck range-of-motion. Approximately 12 hours later, she again applied the same heat stimulus to the same trigger points and her pain and movement were completely improved without a return of her symptoms.

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of the appended claims.

Accordingly, the preceding merely illustrates the principles of the invention. It will be appreciated that those skilled in the art will be able to devise various arrangements which, although not explicitly described or shown herein, embody the principles of the invention and are included within its spirit and scope. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present invention, therefore, is not intended to be limited to the exemplary embodiments shown and described herein. Rather, the scope and spirit of present invention is embodied by the appended claims.

## Claims

1. A hand-held heating device for topically administering heat, the device comprising a heat application surface for applying heat to a tender area on the skin surface of an individual.

2. The device according to claim 1, comprising temperature selection means capable of selecting temperature in 1 degree-centigrade increments to within one-half degree centigrade.

3. The device according to claims 1 or 2, comprising means for detecting the temperature of the user's skin surface during heat application, the detecting means preferably taking the form of a transducer or thermostat arranged adjacent the heat application surface, which heat application surface is preferably displaceable between an extended skin heating position and a retracted position.

4. The device according to any of the preceding claims, comprising a self-contained power supply.

5. The device according to any of the preceding claims, comprising control means for controlling the temperature of the heat application surface.

6. The device according to claim 5, wherein the control means comprise control electronics which supply current to the heat application surface when the temperature sensed by the temperature detecting means is below the temperature commanded by the temperature selector, and which stop the current supply when the temperature sensed by the temperature detecting means has reached the temperature commanded by the temperature selector.

7. The device according to any of the preceding claims, further comprising a power-on light indicator.

8. The device according to any of the preceding claims, further comprising light indicators which illuminate once a pre-selected heat application temperature has been reached.

9. A method for controlling the temperature of a heat application surface comprised in a device according to any of the preceding claims, the method comprising the steps of: activating the device, placing the device on a skin surface, sensing the temperature of the skin surface, selecting the temperature of the heat application surface and automatically adjusting the temperature of the heat application surface in response to the temperature detecting means.

10. A kit comprising a device according to any of the preceding claims 1-8, and instructions for applying heat using the device to a tender area of a skin surface of a human.

11. A method for treating a subject for non-neuropathic pain, said method comprising applying focused heat to a tender area of a skin surface of said subject in a manner sufficient to treat said subject for said non-neuropathic pain with an optionally disposeable device or kit according to any of the preceding claims 1-8, 10.
